(19) Europäisches Patentamt
European Patent Office
Office européen des brevets

(11) Publication number: 0 033 554
B1

(12) EUROPEAN PATENT SPECIFICATION

(45) Date of publication of patent specification: 09.11.83

(51) Int. Cl.³: C 07 C 45/50, C 07 C 47/02, C 07 C 47/21, B 01 J 31/24

(21) Application number: 81200033.9

(22) Date of filing: 12.01.81

# (54) A process for the hydroformylation of conjugated dienes.

(30) Priority: 04.02.80 GB 8003617

(43) Date of publication of application:
12.08.81 Bulletin 81/32

(45) Publication of the grant of the patent:
09.11.83 Bulletin 83/45

(84) Designated Contracting States:
DE FR GB NL

(56) References cited:
US - A - 3 239 566
US - A - 3 946 082
US - A - 4 101 588

TETRAHEDRON LETTERS, no. 32, 1969
Pergamon Press (GB) B. Fell et al.: "Dialdehydes by hydroformylation of conjugated dienes" pages 2721-2723

(73) Proprietor: SHELL INTERNATIONALE RESEARCH MAATSCHAPPIJ B.V.
Carel van Bylandtlaan 30
NL-2596 HR Den Haag (NL)

(72) Inventor: van Leeuwen, Petrus W. N. M.
Badhuisweg 3
NL-1031 CM Amsterdam (NL)
Inventor: Roobeek, Cornelis Franciscus
Badhuisweg 3
NL-1031 CM Amsterdam (NL)

(74) Representative: Puister, Antonius Tonnis, Mr. et al,
P.O. Box 302
NL-2501 CH The Hague (NL)

EP 0 033 554 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Process for the hydroformylation of conjugated dienes

Hydroformylation of conjugated dienes with the aid of rhodium catalysts is known. For instance, in the Journal of Molecular Catalysis, 2 (1977), pp. 211—218 it is described that a conjugated diene may be hydroformylated under high pressure with a rhodium catalyst and a tertiary phosphine as a co-catalyst. Dependent on the choice of the co-catalyst used in the hydroformylation the yield of linear saturated mono-aldehyde generally ranges from about 30 to 60%, based on the conjugated diene. A number of by-products, such as di-aldehydes, unsaturated mono-aldehydes and high-boiling compounds are formed.

This category of prior published literature also encompasses U.S. patent specification 3,239,566, which discloses a diene hydroformylation process using a complex of ruthenium or rhodium in combination with carbon monoxide and a trialkylphosphine.

Applicant has now found a process for the hydroformylation of conjugated dienes wherein the content of the by-products is suppressed and the amount of linear product is considerably improved.

In this connection mention should be made of British patent specification 988,941, which discloses a hydroformylation process wherein an olefin is reacted with carbon monoxide and hydrogen in the presence of a complex containing in the molecule a transition metal having an atomic number from 23 to 32, 40 to 51 or 57 to 84 and at least one molecule of a biphyllic ligand containing trivalent phosphorus, arsenic or antimony as a catalyst. Although according to the description polydentate phosphorus-containing ligands are disclosed as co-catalysts, no conclusions could be drawn that with the catalyst used in the process according to the present invention still better results in selectivity to linear products could be obtained, since when mono-unsaturated compounds are replaced by conjugated dienes a higher amount of branched products is expected. It has further been proved that a polydentate phosphorus-containing ligand, having aliphatic groups bound to P, has no activity at all. Moreover, in the specification all examples relate to the use of cobalt as catalyst metal in the complex, and no special attention is drawn to rhodium.

Nearest to the process of the present invention, however, is the prior published U.S. patent specification 3,946,082, which discloses a number of specific features of the present process as mentioned in the introductory part of claim 1 but does emphatically restrict its teaching to, as far as the rhodium is concerned, the use of zero valent rhodium complexes (see, for instance, col. 2 lines 14—31). The disadvantages of monovalent rhodium complexes as mentioned in this document appear to be unjustified, however, is shown by the present invention as specifically described hereinafter, as it clearly gives the important advantages as mentioned above in the second paragraph of this description.

Accordingly, the invention provides a process for the hydroformylation of a conjugated diene by reacting a conjugated diene with carbon monoxide and hydrogen in the presence of a complex of rhodium with a polydentate ligand containing trivalent phosphorus atoms bearing substituted or unsubstituted aryl groups and in which ligand any of two phosphorus atoms are separated by a chain of two carbon atoms, characterized in that the rhodium atom or atoms are in the monovalent state.

The polydentate ligand used in the process according to the invention preferably contains substituted or unsubstituted phenyl groups bound to trivalent P. Suitable substituents bound to the phenyl group comprise alkyl groups, alkoxy groups and halogen atoms.

Examples of polydensate ligands are those having one of the following chemical formulae, in which Ø means a phenyl group:

$$Ø_2—P—(CH_2)_2—PØ_2,\ Ø—P[(CH_2)_2—PØ_2]_2,$$

$$P[(CH_2)_2—PØ_2]_3,\ Ø_2P—HC{=}CH—PØ_2,\ \text{and}$$

$$\begin{array}{l} Ø—P—CH—CH_2—PØ_2 \\ \qquad\quad\ | \\ \qquad\quad CH_2 \end{array}$$

Generally, the hydroformylation according to the invention is carried out under rather mild conditions. Preferred reaction temperatures are in the range of from 50° to 200°C and more preferred temperatures from 80° to 130°C and preferred pressures (as the sum of the pressure of hydrogen and carbon monoxide) range of from 3 to 35 bars.

Generally, the molar ratio of hydrogen to carbon monoxide is in the range of from 1 to 12.

The amount of catalyst may vary within wide ranges but generally the molar ratio of catalyst to diene is in the range of from 1:10,000 to 1:10. The catalyst may be built up from 1,5-cyclooctadiene-rhodium(I) acetate or another rhodium source. (1,5-Cyclooctadiene-rhodium(I) acetate occurs in the form of the dimer. In the Examples the calculations were made as if it is a monomer).

The hydroformylation of the conjugated dienes is generally carried out in a hydrocarbon solvent. Preferred solvents are toluene, benzene, cyclohexane or methylcyclohexane.

Examples of conjugated dienes used in the process according to the present invention are 1,3-butadiene, isoprene, 1,3-pentadiene, and conjugated dienes with more than 5 carbon atoms per molecule.

The following Examples further illustrate the invention.

### Example 1A

A 100 ml stainless steel autoclave was charged with a solution of 0.02 mmol of 1,5-cyclooctadiene-rhodium(I) acetate and 0.1 mmol of 1,2-bis(diphenylphosphino) ethane in 20 ml toluene and with 20 mmol of freshly distilled 1,3-butadiene under an atmosphere of argon. The autoclave was then pressurized with an equimolar mixture of hydrogen and carbon monoxide and heated to 120°C, the pressure in the autoclave being then 12 bars. After 3 h at 120°C, the pressure had dropped to 9 bars. The autoclave was then cooled to ambient temperature and the gases were flashed off at atmospheric pressure. A pale yellow liquid remained in the autoclave. This liquid was analyzed by gas chromatography and the constituents were identified by gas chromatography and nuclear magnetic resonance spectroscopy by comparison with authentic samples.

The conversion of 1,3-butadiene was 75%, the selectivity to pentanals was 90%, of which 99% was n-pentanal.

The by-product, a mixture of pentanals, was formed in an amount of only 5%, the remaining 5% were dialdehydes, which were not further identified.

A number of Examples 1B—5 are given with different polydentate ligands, using the same method as described above. A number of comparative experiments A—F is also given. Unlike otherwise stated in the Table the reaction conditions are the same as those applied in Example 1A. The designation Ø in the chemical formulae means a phenyl group.

TABLE

| Example | Ligand | P/Rh atom ratio | Reaction temp., °C | % selectivity to pentanals | % linearity of pentanals | turnover mol. $C_4$/mol. Rh.h |
|---|---|---|---|---|---|---|
| 1A<br>1B | $Ø_2P(CH_2)_2PØ_2$ | 10<br>4 | 120<br>95 | 90<br>>80 | 99<br>98 | 250<br>90 |
| 2 | 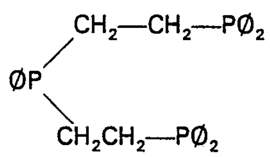 | 3 | 120 | >80 | 98 | 120 |
| 3 | 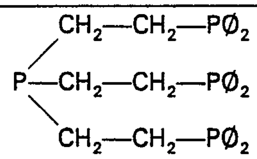 | 4 | 120 | >80 | 98 | 90 |
| 4 | $Ø_2P—CH{=}CH—PØ_2$ | 10 | 120 | 80 | 98 | 150 |
| 5 | 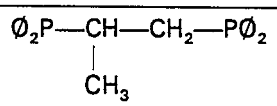 | 4 | 95 | 90 | 98 | 200 |
| Comparative Experiment A | $Ø_2P(CH_2)_4PØ_2$ | 4 | 95 | <20 | 80 | 80 |

TABLE (continued)

| Example | Ligand | P/Rh atom ratio | Reaction temp., °C | % selectivity to pentanals | % linearity of pentanals | turnover mol. $C_4$/mol. Rh.h |
|---|---|---|---|---|---|---|
| Comparative Experiment B | $\O_2P(CH_2)_3P\O_2$ | 4 | 95 | 20 | — | 30 |
| C | $P\O_3$ | 10 | 120 | — | — | <6 |
| D | $\O_2P(CH_2)_{10}P\O_2$ | 10 | 120 | 20 | — | 75 |
| E | $\O_2PCH_2P\O_2$ | 10 | 120 | — | — | <3 |
| F | $(CH_3)_2P(CH_2)_2P(CH_3)_2$ | 10<br>2 | 120<br>120 | —<br>— | —<br>— | <10<br><10 |

From the Table the following may be concluded:

The presence of a chain of two carbon atoms, which are bound to P-atoms is essential since a chain of one, three, four or ten carbon atoms gives a low conversion and a low selectivity to n-pentanal. There is hardly any conversion using $P\O_3$ as a ligand. The presence of a phenyl group is also essential, since when phenyl is replaced by an alkyl group, e.g. methyl group, the conversion is extremely low, notwithstanding the presence of a chain of two carbon atoms.

Example 6

A 100 ml stainless steel autoclave was charged with a solution of 1,5-cyclo-octadiene-rhodium(I) acetate (0.02 mmol.) and 1,2-bis(diphenylphosphino)ethane (0.10 mmol.) in benzene (20 ml) and with isoprene (20 mmol.) under an atmosphere of argon. The autoclave was then pressurized to 12 bars with an equimolar mixture of hydrogen and carbon monoxide and heated to 120°C. After 3 hours at 120°C the autoclave was cooled to ambient temperature and the gases were flashed off at atmospheric pressure. Analysis of the liquid revealed an almost quantitative conversion of isoprene with a selectivity to 3-methylpentanal of 38%. The turnover was 400 mmol. of isoprene/Rh.h.

**Claims**

1. A process for the hydroformylation of a conjugated diene by reacting a conjugated diene with carbon monoxide and hydrogen in the presence of a complex of rhodium with a polydentate ligand containing trivalent phosphorus atoms bearing substituted or unsubstituted aryl groups and in which ligand any of two phosphorus atoms are separated by a chain of two carbon atoms, characterized in that the rhodium atom or atoms are in the monovalent state.

2. A process as claimed in claim 1, characterized in that the polydentate ligand has the formula:

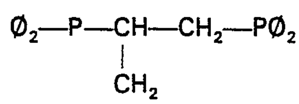

3. A process as claimed in claim 1, characterized in that the polydentate ligand has the formula:

$$\O_2—P—CH{=}CH_2—P\O_2$$

4. A process as claimed in any one of claims 1 to 3, characterized in that the molar ratio of hydrogen to carbon monoxide is in the range of 1 to 12.

**Revendications**

1. Un procédé pour l'hydroformylation d'un diène conjugué en faisant réagir un diène conjugué avec l'oxyde de carbone et l'hydrogène en présence d'un complexe de rhodium avec un ligand polydenté contenant des atomes de phosphore trivalent portant des groupes aryle substitués ou non, ligand dans lequel deux atomes de phosphore quelconques sont séparés par une chaîne de deux atomes de carbone, caractérisé en ce que l'atome ou les atomes de rhodium sont à l'état monovalent.

2. Un procédé selon la revendication 1, caractérisé en ce que le ligand polydenté a la formula:

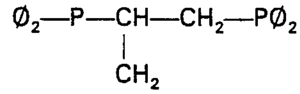

3. Un procédé selon la revendication 1, caractérisé en ce que le ligand polydenté a la formule:

$$\emptyset_2—P—CH{=}CH—P\emptyset_2$$

4. Un procédé selon l'une quelconque des revendications 1 à 3, caractérisé en ce que le rapport molaire de l'hydrogène à l'oxyde de carbone est compris entre 1 et 12.

**Patentansprüche**

1. Ein Verfahren zur Hydroformylierung eines konjugierten Diens durch Umsetzen eines konjugierten Diens mit Kohlenmonoxid und Wasserstoff in Anwesenheit eines Komplexes von Rhodium mit einem mehrzähnigen Liganden, der 3-wertige Phosphoratome enthält, welche substituierte oder nichtsubstituierte Arylgruppen tragen, und in welchem Liganden beliebige von zwei Phosphoratomen durch eine Kette von zwei Kohlenstoffatomen voneinander getrennt sind, dadurch gekennzeichnet, daß das oder die Rhodiumatom(e) im einwertigen Zustand vorliegen.

2. Ein Verfahren wie in Anspruch 1 beansprucht, dadurch gekennzeichnet, daß der mehrzähnige Ligand die nachstehende Formel hat:

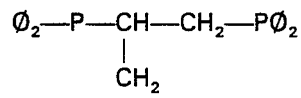

3. Ein Verfahren wie in Anspruch 1 beansprucht, dadurch gekennzeichnet, daß der mehrzähnige Ligand die nachstehende Formel hat:

$$\emptyset_2—P—CH{=}CH—P\emptyset_2$$

4. Ein Verfahren wie in irgendeinem der Ansprüche 1 bis 3 beansprucht, dadurch gekennzeichnet, daß das molare Verhältnis von Wasserstoff zu Kohlenstoff im Bereich von 1 bis 12 liegt.